(19) Europäisches Patentamt
European Patent Office
Office européen des brevets

(11) Veröffentlichungsnummer: **0 604 852 A1**

# (12) EUROPÄISCHE PATENTANMELDUNG

(21) Anmeldenummer: **93120374.9**

(22) Anmeldetag: **17.12.93**

(51) Int. Cl.[5]: **C07C 311/59**, C07C 311/64, C07C 311/58, C07C 311/60, C07C 335/42, C07D 213/70, C07D 295/20, A61K 31/64, A61K 31/18

(30) Priorität: **28.12.92 DE 4244318**

(43) Veröffentlichungstag der Anmeldung: **06.07.94 Patentblatt 94/27**

(84) Benannte Vertragsstaaten: **AT BE CH DE DK ES FR GB GR IE IT LI LU NL PT SE**

(71) Anmelder: **HOECHST AKTIENGESELLSCHAFT**
**Brüningstrasse 50**
**D-65929 Frankfurt am Main(DE)**

(72) Erfinder: **Dr. Weichert, Andreas**
**Rauenthaler Weg 11**
**D-60529 Frankfurt/Main(DE)**
Erfinder: **Dr. Mauger, Jacques**
**72 Bd de Port Royal**
**F-75005 Paris(FR)**
Erfinder: **Dr. Lang, Hans-Jochen**
**Rüdesheimer Strasse 7**
**D.65719 Hofheim/Taunus(DE)**
Erfinder: **Dr. Scholz, Wolfgang**
**Unterortstrasse 30**
**D-65760 Eschborn(DE)**
Erfinder: **Dr. Albus, Udo**
**Am Römerkastell 9**
**D-61197 Florstadt(DE)**

## (54) 2,4-Substituierte 5-(N-substituierte-Sulfamoyl)-Benzoylguanidine, als Antiarrythmika, Inhibitoren der Proliferationen von Zellen, und Inhibitoren des Natrium-Protonen-Antiporters.

**Beschrieben werden Benzoylguanidine der Formel**

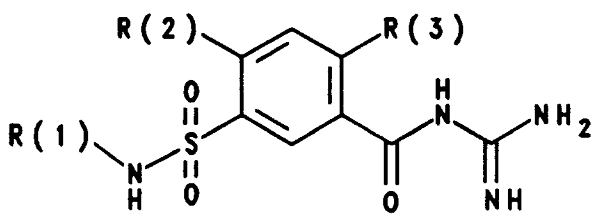

I

(57) worin sind:

R(1) gleich R(4)R(5)N-C(X)- mit X gleich O, S oder N-R(6), R(4) und R(5) gleich H, Alk(en)yl(-R(7)), R(7) gleich Cycloalkyl, Phenyl, oder R(4) und R(5) gemeinsam 4 oder 5 Methylengruppen, R(6) wie R(4) oder gleich Amidin,

R(2) gleich H, Hal, (Cyclo)Alk(en)(in)yl(alkyl), Phenyl(alkyl), Aromat, Heterocyclus, oder -W-R(8), mit W gleich Sauerstoff, S, NR(9), 8

R(3): H, Hal, Alkyl oder -X-R(8) wie für R(2),

sowie deren pharmazeutisch akzeptable Salze.

Sie werden durch Umsetzen einer Verbindung der Formel II

EP 0 604 852 A1

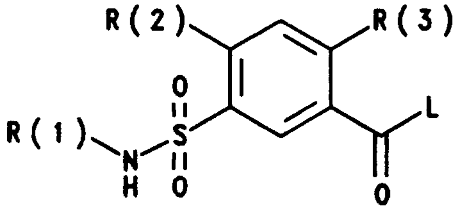

II

mit Guanidin erhalten.

Die Erfindung betrifft Benzoylguanidine der Formel I.

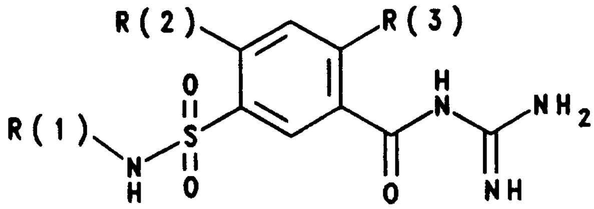

I

worin bedeuten

R(1): R(4)R(5)N-C(X)- mit
X gleich Sauerstoff, S oder N-R(6),
R(4) und R(5) gleich oder verschieden, H, $(C_1-C_8)$-Alkyl, $(C_3-C_6)$-Alkenyl, $-C_nH_{2n}$-R(7),
n gleich Null, 1, 2, 3 oder 4,
R(7) gleich $(C_5-C_7)$-Cycloalkyl, Phenyl, welches unsubstituiert ist oder substituiert mit 1 - 3 Substituenten aus der Gruppe F, Cl, $CF_3$, Methoxy oder $(C_1-C_4)$-Alkyl, oder
R(4) und R(5) gemeinsam 4 oder 5 Methylengruppen, von denen eine $CH_2$-Gruppe durch Sauerstoff, S, NH, N-$CH_3$ oder N-Benzyl ersetzt sein kann, wobei
R(6) wie R(4) definiert ist oder gleich Amidin,

R(2): H, F, Cl, Br, I, $(C_1-C_8)$-Alkyl, 1-Alkenyl oder 1-Alkinyl, $(C_3-C_8)$-Cycloalkyl, $(C_3-C_8)$-Cycloalkyl-$(C_1-C_4)$-Alkyl, Phenyl, $C_6H_5$-$(C_1-C_4)$-Alkyl, Naphthyl, Biphenylyl, 1,1-Diphenyl-$(C_1-C_4)$-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8), mit
W gleich Sauerstoff, S oder NR(9),
R(8) gleich H, $(C_1-C_6)$-Alkyl, $(C_5-C_7)$-Cycloalkyl, Cyclohexylmethyl, Cyclopentylmethyl, $-(CH_2)_mC_pF_{2p+1}$, $-C_qH_{2q}$-R(10), mit
m = Null oder 1,
p = 1, 2 oder 3,
q = Null, 1, 2, 3 oder 4,
R(10) gleich Phenyl, das unsubstituiert oder mit 1 - 3 Substituenten aus der Gruppe F, Cl, $CF_3$, Methyl, Methoxy oder NR(11)R(12) mit R(11) und R(12) in der Bedeutung von H, $(C_1-C_4)$-Alkyl substituiert ist,
R(9) gleich H oder $(C_1-C_3)$-Alkyl, wobei
R(8) und R(9) auch gemeinsam 4 oder 5 Methylengruppen sein können und von denen eine $CH_2$-Gruppe durch Sauerstoff, S, NH, N-$CH_3$ oder N-Benzyl ersetzt sein kann,

R(3): H, F, Cl, Br, I, $(C_1-C_6)$-Alkyl oder -W-R(8) wie für R(2) definiert,

sowie deren pharmazeutisch akzeptable Salze.

Bevorzugt sind Verbindungen I, in denen bedeuten:

R(1): R(4)R(5)N-C(X)- mit
X gleich Sauerstoff, S oder N-R(6),
R(4) und R(5), gleich oder verschieden, H, $(C_1-C_8)$-Alkyl, $-C_nH_{2n}$-R(7) mit
n gleich Null, 1 oder 2,
R(7) gleich $(C_5-C_7)$-Cycloalkyl, Phenyl welches unsubstituiert ist oder substituiert mit 1 - 3 Substituenten aus der Gruppe F, Cl, $CF_3$, Methoxy oder $(C_1-C_4)$-Alkyl, oder
R(4) und R(5) gemeinsam 4 oder 5 Methylengruppen, von denen eine $CH_2$-Gruppe durch Sauerstoff, S, NH, N-$CH_3$ oder N-Benzyl ersetzt sein kann,
R(6) wie R(4) definiert ist oder gleich Amidin,

R(2) H, F, Cl, $(C_1-C_8)$-Alkyl, $(C_3-C_8)$-Cycloalkyl, $(C_3-C_8)$-Cycloalkyl-$(C_1-C_2)$-Alkyl, Phenyl, Naphthyl, Biphenylyl, Pyridyl, Thiopyridyl, Pyrrolyl oder -W-R(8) mit
W gleich Sauerstoff, S oder NR(9),
R(8) gleich H, $(C_1-C_6)$-Alkyl, $-(CH_2)_mC_pF_{2p+1}$, $C_qH_{2q}$-R(10), mit
m = Null oder 1,
p = 1, 2 oder 3,
q = Null, 1, 2, 3 oder 4,
R(10) = Phenyl, das unsubstituiert oder mit 1 - 3 Substituenten aus der Gruppe F, Cl, $CF_3$,

Methyl, Methoxy oder NR(11)R(12), mit R(11) und R(12) in der Bedeutung von H oder ($C_1$-$C_4$)-Alkyl, substituiert ist,

R(9) gleich H oder ($C_1$-$C_3$)-Alkyl, wobei

R(8) und R(9) auch gemeinsam 4 oder 5 Methylengruppen sein können und eine $CH_2$-Gruppe durch Sauerstoff, S, NH, N-$CH_3$ oder N-Benzyl ersetzt sein kann,

R(3) H, F, Cl, ($C_1$-$C_3$)-Alkyl oder -W-R(8) wie für R(2) definiert.

Besonders bevorzugt sind Verbindungen der Formel I, in denen bedeuten:

R(1) R(4)R(5)N-C(X)- mit

X gleich Sauerstoff, S oder NR(6),

R(4) und R(5) gleich oder verschieden, H, ($C_1$-$C_4$)-Alkyl, -$C_nH_{2n}$-R(7),

n gleich Null, 1 oder 2,

R(7) gleich ($C_5$-$C_7$)-Cycloalkyl oder

R(4) und R(5) gemeinsam 4 oder 5 Methylengruppen, von denen eine $CH_2$-Gruppe durch Sauerstoff, S, NH, N-$CH_3$ oder N-Benzyl ersetzt sein kann,

wobei R(6) wie R(4) definiert ist oder gleich Amidin,

R(2) Wasserstoff, F, Cl, ($C_1$-$C_4$)-Alkyl, Thiopyridyl oder W-R(8) mit

W gleich Sauerstoff, S oder NR(9),

R(8) ($C_1$-$C_6$)-Alkyl, -$C_qH_{2q}$-R(10) mit

q = Null, 1, 2, 3, 4 und

R(10) gleich Phenyl,

das unsubstituiert oder mit 1 - 3 Substituenten aus den Gruppen F, Cl, $CF_3$, Methyl, Methoxy oder NR(11)R(12), mit R(11) und R(12) in der Bedeutung von H oder ($C_1$-$C_4$)-Alkyl substituiert ist,

R(9) gleich H oder ($C_1$-$C_3$)-Alkyl, oder

R(8) und R(9) gemeinsam 4 oder 5 Methylengruppen, von denen eine $CH_2$-Gruppe durch Sauerstoff, S, NH, N-$CH_3$ oder N-Benzyl ersetzt sein kann,

R(3) H, F, Cl, ($C_1$-$C_3$)-Alkyl,

sowie deren pharmazeutisch verträgliche Salze.

Enthält einer der drei Substituenten R(1), R(2) und R(3) ein Asymmetriezentrum, so gehören sowohl S- als auch R-konfigurierte Verbindungen zur Erfindung. Die Verbindungen können als optische Isomere, als Diastereoisomere, als Racemate oder als Gemische derselben vorliegen.

Alkyl, Alkenyl oder Alkinyl bedeuten, sofern nicht ausdrücklich anders erwähnt, stets Gruppen mit gerader oder verzweigter Kette.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid.

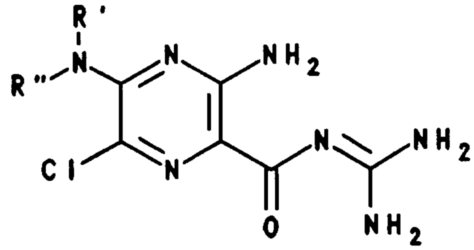

Amilorid: R', R" = H

Dimethylamilorid: R', R" = $CH_3$

Ethylisopropylamilorid: R' = $C_2H_5$, R" = $CH(CH_3)_2$

Darüberhinaus sind Untersuchungen bekannt geworden, die auf antiarrhythmische Eigenschaften von Amilorid hinweisen [Circulation 79, 1257-63 (1989)]. Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten [Eur. Heart J. 9 (Suppl.): 167 (1988) (book of abstracts)]. So wurde beispielsweise

an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 (HOE 89/F 288) sind Benzoylguanidine beschrieben, die in der dem Rest R(1) entsprechenden Stellung ein Wasserstoff tragen. In der deutschen Patentanmeldung P 42 04 575.4 (HOE 92/034) werden 3,5-substituierte Benzoylguanidine vorgeschlagen, in welchen aber die Substituenten R(1) und R(2) nicht die nach der vorliegenden Erfindung beanspruchten Bedeutung haben.

Im US-Patent 3 780 027 werden Acylguanidine beansprucht, die strukturell den Verbindungen der Formel I ähnlich sind. Entscheidender Unterschied zu den Verbindungen I besteht darin, daß es sich um Benzoylguanidine handelt, die sich in ihrem Substitutionsmuster von im Handel befindlichen Diuretika, wie Bumetanid und Furosemid, ableiten und eine für die in diesem Patent angestrebte salidiuretische Wirkung wichtige Aminogruppe in Position 2 bzw. 3 zur Carbonylguanidingruppe tragen. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoff-Mangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen der Formel I infolge Inhibition des zellulären $Na^+/H^+$ Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindung auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen der Formel I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen der Formel I durch starke inhibierende Wirkung auf die Proliferationen von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters ($Na^+/H^+$ -Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindung der Formel I für die präventive Therapie zur Verhinderung der Genese des Bluthochdrucks, beispielweise der essentiellen Hypertonie, geeignet.

Die Erfindung betrifft weiterhin ein Verfahren zur Herstellung der Verbindungen I, dadurch gekennzeichnet, daß man Verbindungen der Formel II

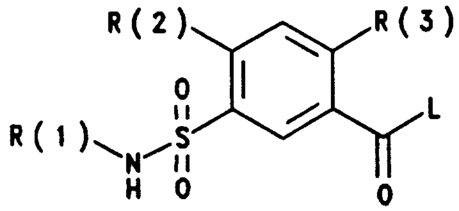

II

mit Guanidin umsetzt, worin R(1) bis R(3) die angegebene Bedeutung besitzen und L für eine leicht nucleophil substituierbare leaving group steht.

Die aktivierten Säurederivate der Formel II, worin L eine Alkoxy-, vorzugsweise eine Methoxygruppe, eine Ethoxygruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH) beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, wie beispielsweise die Methylester oder Ethylester der Formel II mit L = $OCH_3$ oder OEt durch Behandeln mit gasförmigem HCl in Methanol oder Ethanol, die Imidazolide der Formel II durch Behandeln mit Carbonyldiimidazol [L = 1-Imidazolyl; Staab, Angew. Chem. Int. Ed. Engl. 1, 351 - 367 (1962)], die gemischten Anhydride II mit $Cl\text{-}COOC_2H_5$ oder Tosylchlorid in Gegenwart von Triethylamin in einem inerten Lösungsmittel, wie auch die Aktivierungen von Benzoesäuren mit Dicylclohexylcarbodiimid (DCC) oder mit O-[(Cyano(ethoxycarbonyl)-methylen)amino]-1,1,3,3-tetramethyluroniumtetrafluororoborat ("TOTU") [Weiss und Krommer, Chemiker Zeitung 98, 817 (1974)]. Eine Reihe geeigneter Methoden zur Herstellung von aktivieren Carbonsäurederivaten der Formel 11 sind unter Angabe von Quellenliteratur in J. March, Advanced Organic Chemistry, Third Edition (John Wiley & Sons, 1985), S. 350, angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol oder THF zwischen 20 °C und Siedetemperatur dieser Lösungsmittel bewährt. Bei den meisten Umsetzungen von Verbindungen II mit salzfreien Guanidin wurde vorteilhaft in aprotischen inerten Lösungsmitteln wie THF, Dimethoxyethan, Dioxan gearbeitet. Aber auch Wasser kann als Lösungsmittel dienen.

Wenn L = Cl bedeutet, arbeitet man vorteilhaft unter Zusatz eines Säurefängers, z.B. in Form von überschüssigen Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden, indem man beispielsweise 2,4-Dihalogen-5-chlorsulfonylbenzoesäuren mit Ammoniak in 5-Aminosulfonyl-2,4-dihalogenbenzoesäuren (III a, L = OH) überführt und die erhaltenen Benzoesäuren nach einer der oben beschriebenen Verfahrensvarianten zu erfindungsgemäßen Verbindungen I umgesetzt werden.

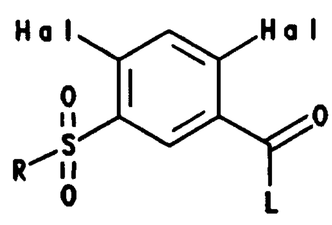

III

IIIa) R = NR(4)R(5)

IIIb) R = R(4)R(5)NC(X)

Derartige Carbonsäuren oder deren Ester der Formel III mit L = -OH oder beispielsweise -O-Methyl oder

-O-Ethyl können aber auch als Ausgangsverbindungen für andere Carbonsäuren dienen, wobei das Halogen in R(2)- und/oder R(3)-Position sehr bequem in bekannter Weise gegen zahlreiche nucleophile Reagenzien, wie Mercaptane R(8)-SH oder Alkohole R(8)-OH oder Amine R(8)R(9)NH ausgetauscht werden kann.

Die Einführung der Alkyl- oder Arylsubstituenten gelingt durch literaturbekannte Methoden des Palladium-vermittelten cross-couplings von Arylhalogeniden mit beispielsweise Organozink- bzw. Kupferverbindungen, Organostannanen, Organoboronsäuren oder Organoboranen.

Die Benzoesäurederivate der Formel III (III b, X = O, S) werden durch die Reaktion der Natriumsalze von IIIa ($R_4$, $R_5$ = H) mit Isocyanaten oder Carbamidsäurechloriden (X = O) oder Isothiocyanaten (X = S) hergestellt. S-Alkylierung von IIIb (X = S) gefolgt vom Austausch mit einem Amin [R(6)$NH_2$] führt zu den Benzoesäurederivaten der Formel IIIb [X= NR(6)].

Benzoylguanidine I sind im allgemeinen schwache Basen und können Säure unter Bildung von Salzen binden. Als Säureadditionssalze kommen Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar in der Veterinär- als auch in der Humanmedizin.

Welche Hilfsstoffe für die gewünschte Arzneimittelformulierung geeignet sind, ist dem Fachmann aufgrund seines Fachwissens geläufig. Neben Lösemitteln, Gelbildnern, Suppositoriengrundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z.B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z.B. in Frage: Wasser, physiologische Kochsalzlösung oder Alkohole, z.B. Ethanol, Propanol, Glyzerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z.B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg, vorzugsweise 0,01 mg bis 10 mg, vorzugsweise 1 mg. Bei akuten Ausbrüchen der Krankheit, etwa unmittelbar nach Erleiden eines Herzinfarkts, können auch noch höhere und vor allem häufigere Dosierungen notwendig sein, z.B. bis zu 4 Einzeldosen pro Tag. Insbesondere bei i.v. Anwendung, etwa bei einem Infarktpatienten auf der Intensivstation können bis zu 100 mg pro Tag notwendig werden.

Experimenteller Teil:

Allgemeine Vorschrift zur Herstellung von Benzoylguanidinen (I) aus Benzoesäuremethylester (oder -ethylester) (II, L = OMe oder OEt) 0.01 M des Benzoesäuremethyl-(oder -ethyl)esterderivates der

allgemeinen Formel II löst bzw. suspendiert man in 60 ml wasserfreiem Tetrahydrofuran (THF) und versetzt dann mit 0.05 M Guanidin. Nachdem einige Stunden zum Rückfluß erhitzt wurde, destilliert man das THF ab, versetzt mit Wasser, stellt mit 2N HCl auf pH 6 - 7 und filtriert das entsprechende Benzoylguanidin (I) ab. Die auf diese Weise erhaltenen Acylguanidine können durch Behandeln mit wäßriger oder methanolischer Salzsäure oder anderen pharmakologisch verträglichen Säuren in die entsprechenden Salze überführt werden.

Beispiel 1:

4-Chlor-3-(N-propyl-carbamoyl-sulfamoyl)benzoylguanidin-hydrochlorid:farblose Kristalle, Schmp. 205 - 207 °C

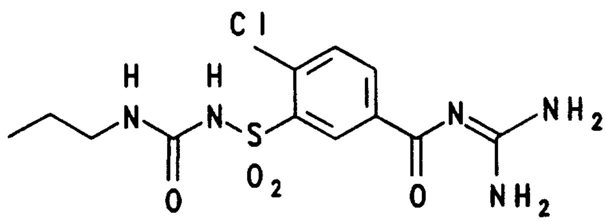

Syntheseweg:

a) 4-Chlor-3-(N-propyl-carbamoyl-sulfamoyl)benzoesäure-methylester. Addition von 4-Chlor-3-sulfamoylbenzoesäuremethylester zu Propylisocyanat mit 2 Äquivalenten Kaliumcarbonat in Aceton (Rückfluß, 1 Stunde), Acidifizieren mit HCl und Abfiltrieren des Niederschlags, farblose Kristalle, Schmp. 185 °C
b) 4-Chlor-3-(N-propyl-carbamoyl-sulfamoyl)benzoyl-guanidin-hydrochlorid
aus a) nach allg. Vorschrift.

Beispiel 2:

4-Chlor-3-(N-ethyl-thiocarbamoyl-sulfamoyl)-benzoyl-guanidin-hydrochlorid: farblose Kristalle, Schmp. 175 °C.

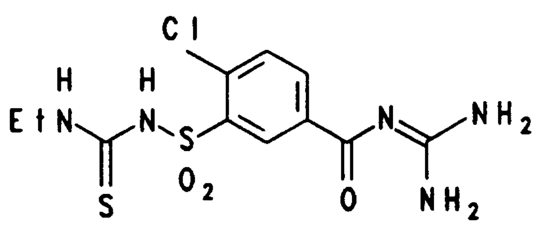

Syntheseweg:

a) 4-Chlor-3-(N-ethyl-thiocarbamoyl-sulfamoyl)-benzoesäure-methylester Addition von 4-Chlor-3-sulfamoyl-benzoesäuremethylester zu Ethylisothiocyanat mit 2 Aquivalenten Kaliumcarbonat in Aceton (Rückfluß, 2,5 Stunden), Acidifizieren mit HCl und Abfiltrieren des Niederschlags, Schmp. 175 °C
b) 4-Chlor-3-(N-ethyl-thiocarbamoyl-sulfamoyl)-benzoyl-guanidin-hydrochlorid
aus a) nach allg. Vorschrift.

Beispiel 3:

3-(N-Cyclohexyl-carbamoyl-sulfamoyl)-4-phenoxy-benzoyl-guanidin-hydrochlorid: farblose Kristalle, Schmp. 231 °C.

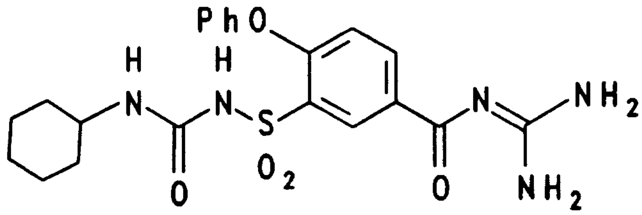

Syntheseweg:

a) 4-Phenoxy-3-suffamoyl-benzoesäure-methylester
Phenol und Kaliumhydroxyd werden zum Sieden erhitzt, bis die Mischung homogen wird, dann mit 4-Fluor-3-sulfamoyl-benzoesäure-methylester versetzt. Nach 45 min. bei 130 ° C wird die Reaktionslösung in Wasser/Eis gegossen und der Niederschlag abfiltriert. Schmp. 145 ° C.
b) 3-(N-Cyclohexyl-carbamoyl-suffamoyl)-4-phenoxy-benzoesäure-methylester
aus a) mit Cyclohexylisocyanat analog Beispiel 1 a), farblose Kristalle, Schmp. 182 ° C.
c) 4-Phenoxy-3-(N-cyclohexyl-carbamoyl-sulfamoyl)benzoyl-guanidin-hydrochlorid
nach allg. Vorschrift aus b).

Beispiel 4:

4-Isopropyl-3-(N-cyclohexyl-carbamoyl-sulfamoyl)-benzoyl-guanidin-hydrochlorid: farblose Kristalle, Schmp. 232 - 236 ° C.

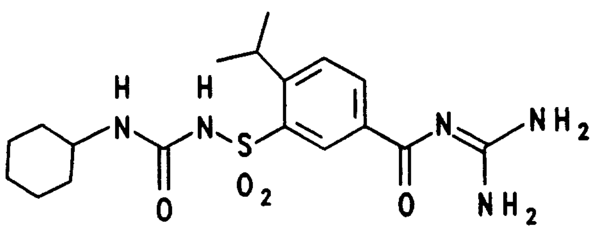

Syntheseweg:

a) 4-Isopropyl-3-suffamoyl-benzoesäure
aus 3-Chlorsulfonyl-4-isopropyl-benzoesäure mit Ammoniak (20 Stunden, Zimmertemperatur), Acidifizieren mit HCl, mit Essigester extrahieren, Lösungsmittel abdestillieren, farblose Kristalle, Schmp. 231 ° C
b) 4-Isopropyl-3-suffamoyl-benzoesäureethylester
aus a) mit HCl/Ethanol-Lösung, Lösungsmittel abdestillieren, Rückstand in Diethylether/Petrolether kristallisieren, farblose Kristalle, Schmp. 110 - 113 ° C.
c) 3-(N-Cyclohexyl-carbamoyl-sufamoyl)-4-isopropyl-benzoesäureethylester
aus b) mit Cyclohexylisocyanat analog 1 a), farbloses Öl.
d) 3-(N-Cyclohexyl-carbamoyl-suffamoyl)-4-isopropyl-benzoyl-guanidin-hydrochlorid
nach allg. Vorschrift.

Beispiel 5:

3-(N-Cyclohexyl-carbamoyl-suffamoyl)-4-(2'-mercapto-pyridinyl)benzoyl-guanidin-hydrochlorid:

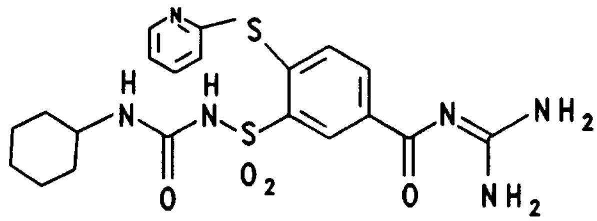

farblose Kristalle, Schmp. 152 - 155 °C

Syntheseweg:

a) 3-Sulfamoyl-4-(2'-mercaptopyridinyl)benzoesäure-ethylester
aus 4-Fluoro-3-sulfamoyl-benzoesäureethylester mit 1 Äquivalent 2-Mercapto-pyridin und 3 Äquivalenten Kaliumcarbonat in DMF (Rückfluß, 6 Stunden), Behandeln mit Wasser, Acidifizieren mit HCl, Extrahieren mit Essigester und Lösungsmittel abdestillieren, Säulenchromatographie an Kieselgel mit Essigester/Methanol (9 : 1). Bräunliche Kristalle, Schmp. 94 - 95 °C
b) 3-(N-Cyclohexyl-carbamoyl-sulfamoyl)-4-(2'-mercapto-pyridinyl)benzoesäureethylester
aus a) mit Cyclohexylisocyanat analog 1 a), farblose Kristalle, Schmp. 145 - 150 °C
c) 3-(N-Cyclohexyl-carbamoyl-sulfamoyl)-4-(2'-mercapto-pyridinyl)benzoyl-guanidin-hydrochlorid
aus b) nach allg. Vorschrift.

Beispiel 6:

2,4-Dichlor-5-(N-phenyl-carbamoyl-sulfamoyl)benzoyl-guanidin-hydrochlorid: farblose Kristalle, Schmp. 215 - 218 °C.

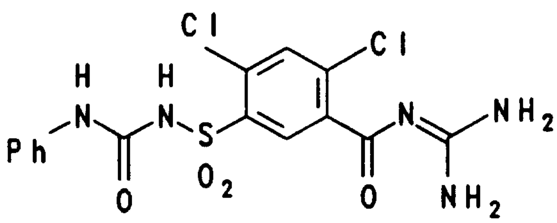

Syntheseweg:

a) 2,4-Dichlor-5-(N-phenyl-carbamoyl-sulfamoyl)-benzoesäure-methylester
aus 2,4-Dichlor-5-sulfamoyl-benzoesäure-methylester und Phenylisocyanat analog 1 a),
farblose Kristalle, Schmp. 187 - 189 °C
b) 2,4-Dichlor-5-(N-phenyl-carbamoyl-sulfamoyl)benzoyl-guanidin-hydrochlorid
aus a) nach allg. Vorschrift I (siehe oben).

Beispiel 7:

5-(N-Cyclohexyl-carbamoyl-suffamoyl)-2,4-dichlor-benzoyl-guanidin-hydrochlorid, farblose Kristalle, Schmp. 211 - 215 °C

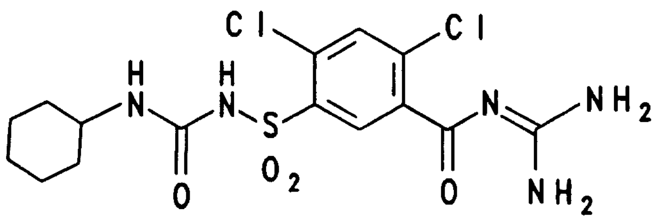

Syntheseweg:

a) 5-(N-Cyclohexyl-carbamoyl-suffamoyl)-2,4-dichlor-benzoesäure-methylester aus 2,4-Dichlor-5-sulfamoyl-benzoesäure-methylester mit Cyclohexylisocyanat analog 1 a), farblose Kristalle, Schmp. 211 - 214 °C.
b) 5-(N-Cyclohexyl-carbamoyl-sulfamoyl)-2,4-dichlor-benzoyl-guanidin-hydrochlorid aus a) nach allg. Vorschrift.

Beispiel 8:

4-Chlor-3-[(N-morpholino-carbonyl)-sulfamoyl]benzoyl-guanidin-hydrochlorid, farblose Kristalle, Schmpkt. 155 °C

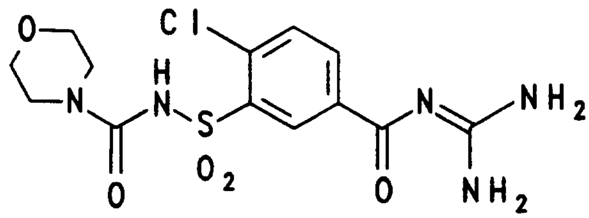

Syntheseweg:

a) 4-Chloro-3-[(N-morpholino-carbonyl)-sulfamoyl]benzoesäure-methylester aus 4-Chloro-3-sulfamoyl-benzoesäure-methylester mit 1,5 Äquivalenten Morpholin-N-carbonsäurechlorid und 2 Äquivalenten Kaliumcarbonat (Rückfluß, 2 Stunden), Behandeln mit Wasser, Acidifizieren mit HCl, Extrahieren mit Essigester und Abdestillieren, farblose Kristalle, Schmp. 162 °C.
b) 4-Chloro-3-[(N-morpholino-carbonyl)sulfamoyl]benzoyl-guanidin-hydrochlorid aus a) nach allg. Vorschrift.

Beispiel 9:

3-[N-(N',N''-Butylethylguanidino)sulfonyl]benzoyl-guanidin-hydrochloridamorph

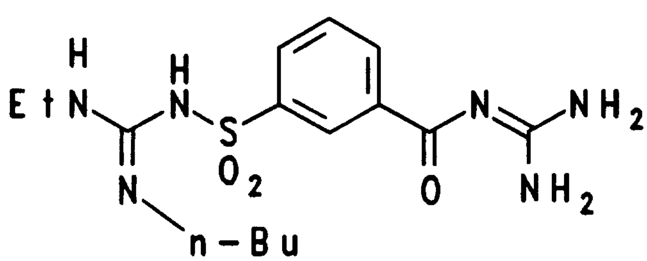

Syntheseweg:

a) 3-[(N-Ethyl-thiocarbamoyl)-sulfamoyl]benzoesäure-methylester
aus 3-Sulfamoyl-benzoesäure-methylester und Ethylisothiocyanat analog 2 a),
farblose Kristalle, Schmp. 126 - 128 °C
b) [(N-Ethyl-S-methyl-thioguanidino-sulfonyl]benzoesäure-methylester
aus a) mit 4 Äquivalenten Methyliodid in Methanol (Rückfluß, 24 Stunden), Lösungsmittel abdestillieren, Rückstand mit Essigester aufnehmen, mit Natriumcarbonat waschen und abdestillieren, Rückstand mit Diisopropylether kristallisieren,
farblose Kristalle, Schmp. 178 - 181 °C
c) 3-[N-(N'-Butyl-N''-Ethyl-guanidino)sulfonyl]benzoesäure-methylester
aus b) mit n-Butylamin (0 °C, 2,5 Stunden), n-Butylamin abdestillieren, Rückstand in Diisopropylether/Essigester kristallisieren.
Schmp. 80 - 85 °C
d) 3-[N-(N'-Butyl-N''-Ethyl-guanidino)sulfonyl]benzoyl-guanidin-hydrochlorid
aus c) nach allg. Vorschrift.

Beispiel 10:

3-[N-(N'-Butyl-N''-Ethylguanidino)sulfonyl]-4-chlor-benzoyl-guanidin-hydrochlorid, farblose Kristalle, Schmp. 212 °C.

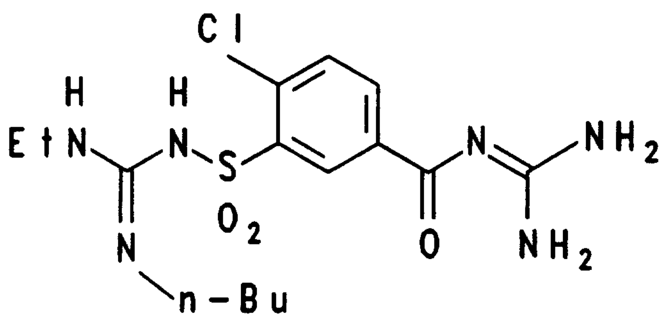

Syntheseweg:

a) 4-Chlor-3-[(N-Ethyl-S-methyl-thioguanidino-sulfonyl]benzoesäure-methylester
aus 2 a) mit Methyliodid analog 9 b),
farblose Kristalle, Schmp. 175 °C
b) 3-[N-(N'-Butyl-N''-Ethyl-guanidino)sulfonyl]-4-chlor-benzoesäure-methylester
aus a) mit n-Butylamin analog 9 c),
farblose Kristalle, Schmp. 82 °C
c) 3-[N-(N'-Butyl-N''-Ethylguanidino)sulfonyl]-4-chlor-benzoyl-guanidin-hydrochlorid
aus b) nach allg. Vorschrift.

Beispiel 11:

4-Chlor-3-[N-(N'-ethyl-N''-amidino)sulfonyl-guanidino]benzoyl-guanidin-hydrochlorid

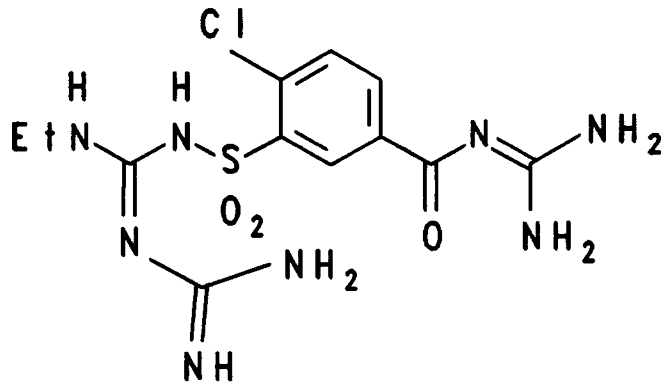

farblose Kristalle, Schmp. 233 - 235 ° C, aus 10 a) mit 10 Äquivalenten Guanidin, nach allg. Vorschrift.

Pharmakologische Daten:

Inhibitoren des $Na^+/H^+$-Exchangers von Kaninchenerythrozyten:

Weiße Neuseeland-Kaninchen (Ivanovas) erhielten eine Standard-Diät mit 2 % Cholesterin für sechs Wochen, um den $Na^+/H^+$-Austausch zu aktivieren und so den $Na^+$-Influx in die Erythrozyten via $Na^+/H^+$-Austausch flammenphotometrisch bestimmen zu können. Das Blut wurde den Ohrarterien entnommen und durch 25 IE/ml Kalium-Heparin ungerinnbar gemacht. Ein Teil jeder Probe wurde zur Doppelbestimmung des Hämatokrits durch Zentrifugation benutzt. Aliquots von jeweils 100 µl dienten zur Messung des $Na^+$-Ausgangsgehalts der Erythrozyten. Um den Amilorid-sensitiven Natrium-Influx zu bestimmen, wurden 100 µl jeder Blutprobe in jeweils 5 ml eines hyperosmolaren Salz-Sucrose-Mediums (mmol/l : 140 NaCl, 3 KCl, 150 Sucrose, 0,1 Ouabain, 20 Tris-hydroxymethyl-aminomethan) bei pH 7,4 und 37 ° C inkubiert. Die Erythrozyten wurden danach dreimal mit eiskalter $MgCl_2$-Ouabain-Lösung (mmol/l : 112 $MgCl_2$, 0,1 Ouabain) gewaschen und in 2,0 ml destilliertem Wasser hämolysiert. Der intrazelluläre Natriumgehalt wurde flammenphotometrisch bestimmt.

Der $Na^+$-Nettoinflux wurde aus der Differenz zwischen Natrium-Ausgangswerten und dem Natriumgehalt der Erythrozyten nach Inkubation errechnet. Der Amiloridhemmbare Natrium-Influx ergab sich aus der Differenz des Natriumgehalts der Erythrozyten nach Inkubation mit und ohne Amilorid 3 x $10^{-4}$ mol/l. Auf diese Weise wurde auch bei den erfindungsgemäßen Verbindungen verfahren.

Ergebnisse

Inhibition des $Na^+/H^+$-Exchangers:

| Beispiel | $IC_{50}$ (mol/l) |
|---|---|
| 2 | 1 x $10^{-5}$ |
| 6 | 2 x $10^{-5}$ |
| 7 | 2 x $10^{-5}$ |

## Patentansprüche

**1.** Benzoylguanidine der Formel I.

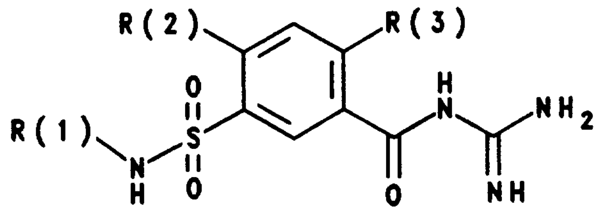

I

worin bedeuten

R(1): R(4)R(5)N-C(X)- mit

X gleich Sauerstoff, S oder N-R(6),

R(4) und R(5), gleich oder verschieden, H, $(C_1\text{-}C_8)$-Alkyl, $(C_3\text{-}C_6)$-Alkenyl, $-C_nH_{2n}$-R(7),

n gleich Null, 1, 2, 3 oder 4,

R(7) gleich $(C_5\text{-}C_7)$-Cycloalkyl, Phenyl,

welches unsubstituiert ist oder substituiert mit 1 - 3 Substituenten aus der Gruppe F, Cl, $CF_3$, Methoxy oder $(C_1\text{-}C_4)$-Alkyl, oder

R(4) und R(5) gemeinsam 4 oder 5 Methylengruppen, von denen eine $CH_2$-Gruppe durch Sauerstoff, S, NH, N-$CH_3$ oder N-Benzyl ersetzt sein kann, wobei

R(6) wie R(4) definiert ist oder gleich Amidin,

R(2): H, F, Cl, Br, I, $(C_1\text{-}C_8)$-Alkyl, 1-Alkenyl oder 1-Alkinyl, $(C_3\text{-}C_8)$-Cycloalkyl, $(C_3\text{-}C_8)$-Cycloalkyl-$(C_1\text{-}C_4)$-Alkyl, Phenyl, $C_6H_5$-$(C_1\text{-}C_4)$-Alkyl, Naphthyl, Biphenylyl, 1,1-Diphenyl-$(C_1\text{-}C_4)$-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8), mit

W gleich Sauerstoff, S oder NR(9),

R(8) gleich H, $(C_1\text{-}C_6)$-Alkyl, $(C_5\text{-}C_7)$-Cycloalkyl, Cyclohexylmethyl, Cyclopentylmethyl, $-(CH_2)_mC_pF_{2p+1}$,$-C_qH_{2q}$-R(10),

m = Null oder 1,

p = 1, 2 oder 3,

q = Null, 1, 2, 3 oder 4,

R(10) gleich Phenyl, das unsubstituiert oder mit 1 - 3 Substituenten aus der Gruppe F, Cl, $CF_3$, Methyl, Methoxy oder NR(11)R(12) mit R(11) und R(12) in der Bedeutung von H, $(C_1\text{-}C_4)$-Alkyl substituiert ist,

R(9) H oder $(C_1\text{-}C_3)$-Alkyl, wobei

R(8) und R(9) auch gemeinsam 4 oder 5 Methylengruppen sein können, von denen eine $CH_2$-Gruppe durch Sauerstoff, S, NH, N-$CH_3$ oder N-Benzyl ersetzt sein kann,

R(3): H, F, Cl, Br, I, $(C_1\text{-}C_6)$-Alkyl oder -W-R(8) wie für R(2) definiert,

sowie deren pharmazeutisch akzeptable Salze.

**2.** Verbindung I nach Anspruch 1, dadurch gekennzeichnet, daß darin bedeuten:

R(1): R(4)R(5)N-C(X)- mit

X gleich Sauerstoff, S oder N-R(6),

R(4) und R(5), gleich oder verschieden, H, $(C_1\text{-}C_8)$-Alkyl, $-C_nH_{2n}$-R(7)

n gleich Null, 1 oder 2,

R(7) gleich $(C_5\text{-}C_7)$-Cycloalkyl, Phenyl,

welches unsubstituiert ist oder substituiert mit 1 - 3 Substituenten aus der Gruppe F, Cl, $CF_3$, Methyl, Methoxy oder $(C_1\text{-}C_4)$-Alkyl, oder

R(4) und R(5) gemeinsam 4 oder 5 Methylengruppen, von denen eine $CH_2$-Gruppe durch Sauerstoff, S, NH, N-$CH_3$ oder N-Benzyl ersetzt sein kann,

R(6) wie R(4) definiert ist oder gleich Amidin,

R(2) H, F, Cl, $(C_1\text{-}C_8)$-Alkyl, $(C_3\text{-}C_8)$-Cycloalkyl, $(C_3\text{-}C_8)$-Cycloalkyl-$(C_1\text{-}C_2)$-Alkyl, Phenyl, Naphthyl, Biphenylyl, Pyridyl, Thiopyridyl, Pyrrolyl oder -W-R(8) mit

W gleich Sauerstoff, S oder NR(9),

R(8) gleich H, $(C_1\text{-}C_6)$-Alkyl, $-(CH_2)_mC_pF_{2p+1}$, $C_qH_{2q}$-R(10), mit

m = Null oder 1,

p = 1, 2 oder 3,

q = Null, 1, 2, 3 oder 4,

R(10) = Phenyl,
das unsubstituiert oder mit 1 - 3 Substituenten aus der Gruppe F, Cl, $CF_3$, Methyl, Methoxy oder NR(11)R(12), mit R(11) und R(12) in der Bedeutung von H oder ($C_1$-$C_4$)-Alkyl, substituiert ist, wobei
R(9) gleich H oder ($C_1$-$C_3$)-Alkyl,
R(8) und R(9) auch gemeinsam 4 oder 5 Methylengruppen sein können und von denen eine $CH_2$-Gruppe durch Sauerstoff, S, NH, N-$CH_3$ oder N-Benzyl ersetzt sein kann,

R(3) H, F, Cl, ($C_1$-$C_3$)-Alkyl oder -W-R(8) wie für R(2) definiert.

**3.** Verbindung I nach Anspruch 1, worin bedeuten:

R(1) R(4)R(5)N-C(X)- mit
X gleich Sauerstoff, S oder NR(6),
R(4) und R(5), gleich oder verschieden, H, ($C_1$-$C_4$)-Alkyl, -$C_nH_{2n}$-R(7),
n gleich Null, 1 oder 2,
R(7) gleich ($C_5$-$C_7$)-Cycloalkyl oder
R(4) und R(5) gemeinsam 4 oder 5 Methylengruppen, von denen eine $CH_2$-Gruppe durch ein Sauerstoff, S, NH, N-$CH_3$ oder N-Benzyl ersetzt sein kann,
wobei R(6) wie R(4) definiert ist oder gleich Amidin,

R(2) Wasserstoff, F, Cl, ($C_1$-$C_4$)-Alkyl, Thiopyridyl oder W-R(8) mit
W gleich Sauerstoff, S oder NR(9),
R(8) ($C_1$-$C_6$)-Alkyl, -$C_qH_{2q}$-R(10) mit
q = Null, 1, 2, 3 oder 4,
R(10) gleich Phenyl,
das unsubstituiert oder mit 1 - 3 Substituenten aus der Gruppe F, Cl, $CF_3$, Methyl, Methoxy oder NR(11)R(12), mit R(11) und R(12) in der Bedeutung von H oder ($C_1$-$C_4$)-Alkyl substituiert ist, oder
R(9) gleich H oder ($C_1$-$C_3$)-Alkyl, oder
R(8) und R(9) gemeinsam 4 oder 5 Methylengruppen, von denen eine $CH_2$-Gruppe durch Sauerstoff, S, NH, N-$CH_3$ oder N-Benzyl ersetzt sein kann,

R(3) H, F, Cl, ($C_1$-$C_3$)-Alkyl.

**4.** Verfahren zur Herstellung einer Verbindung I nach Anspruch 1,
dadurch gekennzeichnet, daß man
eine Verbindung der Formel II

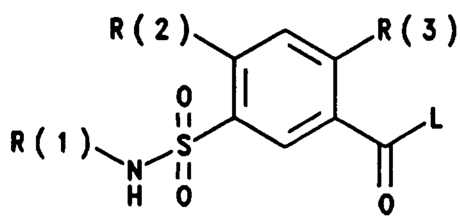

II

worin R(1) bis R(3) die angegebene Bedeutung besitzen und L für eine leicht nucleophil substituierbare leaving group steht,
mit Guanidin umsetzt.

**5.** Verwendung einer Verbindung I nach Anspruch 1 zur Herstellung eines Medikaments zur Behandlung von Arrhythmien.

**6.** Verwendung einer Verbindung I nach Anspruch 1 zur Herstellung eines Medikaments zur Behandlung oder Prophylaxe des Herzinfarkts.

**7.** Verwendung einer Verbindung I nach Anspruch 1 zur Herstellung eines Medikaments zur Behandlung der Angina pectoris.

8. Verwendung einer Verbindung I nach Anspruch 1 zur Herstellung eines Medikaments zur Behandlung von ischämischen Zuständen des Herzens.

9. Verwendung einer Verbindung I nach Anspruch 1 zur Herstellung eines Medikaments zur Behandlung von Krankheiten, bei denen die Fibroblastenproliferation eine primäre oder sekundäre Ursache darstellt, und somit ihre Verwendung als Antiatherosklerotika, Mittel gegen diabetische Spätkomplikationen, Krebserkrankungen, fibrotische Erkrankungen wie Lungenfibrose, Leberfibrose oder Nierenfibrose.

10. Verwendung einer Verbindung I nach Anspruch 1 zur Herstellung eines wissenschaftlichen Tools zur Inhibition des $Na^+/H^+$-Exchangers zur Diagnose der Hypertonie und proliferativer Erkrankungen.

Europäisches Patentamt

# EUROPÄISCHER RECHERCHENBERICHT

Nummer der Anmeldung
EP 93 12 0374

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| A<br><br>D | EP-A-0 416 499 (HOECHST)<br>* Seite 3 *<br>& US-A-5 091 394<br>----- | 1,5-8 | C07C311/59<br>C07C311/64<br>C07C311/58<br>C07C311/60<br>C07C335/42<br>C07D213/70<br>C07D295/20<br>A61K31/64<br>A61K31/18 |

| RECHERCHIERTE SACHGEBIETE (Int.Cl.5) |
|---|
| C07C |

Der vorliegende Recherchenbericht wurde für alle Patentansprüche erstellt

| Recherchenort | Abschlußdatum der Recherche | Prüfer |
|---|---|---|
| DEN HAAG | 18. März 1994 | English, R |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)